# EUROPÄISCHE PATENTSCHRIFT

(11) **EP 0 512 539 B1**
(45) Veröffentlichungstag und Bekanntmachung des Hinweises auf die Patenterteilung: **10.08.1994**
(21) Anmeldenummer: 92107702.0
(22) Anmeldetag: 07.05.1992
(51) Int. Cl.: B67B 1/08, B67B 3/26

(54) **Inspektionsmaschine für Bügelverschlussflaschen**
Inspection equipment for swing-stopper bottles
Dispositif pour l'inspection de bouteilles munis de fermeture à étrier

(30) Priorität: 10.05.1991 DE 4115264; 07.04.1992 DE 4211660
(43) Veröffentlichungstag der Anmeldung: 11.11.1992
(73) Patentinhaber: Zodrow, Rudolf, D-40235 Düsseldorf (DE)
(72) Erfinder: Zodrow, Rudolf, D-40235 Düsseldorf (DE)
(74) Vertreter: Cohausz & Florack Patentanwälte

(56) Entgegenhaltungen:
- DE-A- 3 923 670

## Beschreibung

Die Erfindung bezieht sich auf eine Inspektionsmaschine für Bügelverschlußflaschen, bestehend aus einem Rundläufer mit an seinem Umfang angeordneten Aufnahmeplätzen, in denen die Flaschen zwischen einem drehgesteuerten Drehteller und einem Spannkopf axial eingespannt sind, und mit Funktionselementen für die Ausrichtung der Flaschen bezüglich ihrer Drehstellung sowie mit an die Bügel der Bügelverschlüsse ansetzbaren Hubelementen, mit denen im Bereich von am Weg der in den Aufnahmeplätzen gehaltenen Flaschen die Verschlußstöpsel der Bügelverschlüsse aus ihrer Lage am Flaschenhals in eine angehobene, freihängende Lage überführbar sind, in der sie durch Prüfmittel der Prüfeinrichtung überprüfbar sind.

Bevor die gereinigten Bügelverschlußflaschen mit einem Getränk gefüllt werden, ist es erforderlich, sie auf ihre Sauberkeit und Funktionstüchtigkeit zu überprüfen. Bei einer bekannten Maschine der eingangs genannten Art, die der Füllmaschine nachgeordnet ist, wird lediglich überprüft, ob der Verschlußstöpsel des Bügelverschlusses ordnungsgemäß mit einer Dichtung ausgerüstet ist. Fehlt die Dichtung, dann wird diese Flasche aus der Maschine ausgeschleust. Damit diese Überprüfung möglich ist, wird mittels eines den Flaschenhals gabelförmig umgreifenden Elementes der untere Bügel hochgeschwenkt, so daß der Verschlußstöpsel frei pendelnd den Prüfmitteln präsentiert wird. Diese Art der Überprüfung ist nicht sicher genug, weil der frei pendelnd gehaltene Verschlußstöpsel keine exakt vorbestimmte Position einnimmt, insbesondere sich auf dem Unterbügel verklemmen kann und dann die Dichtung den Prüfmitteln nicht zugekehrt ist. Dies gilt insbesondere unter Berücksichtigung der Tatsache, daß sich die Flaschen bei der Überprüfung nicht in Ruhe befinden, sondern im Drehtisch weiter transportiert werden, so daß die Verschlußstöpsel pendeln können. Neben dieser unsicheren Überprüfung besteht ein weiterer Nachteil bei der bekannten Inspektionsmaschine darin, daß weitere Prüfungen der Bügelverschlußflasche nicht vorgesehen sind (DE-A-3 923 670).

Bei Inspektionsmaschinen für andere Flaschen, z.B. Schraubverschlußflaschen oder Kronkorkenflaschen umfassen die Prüfeinrichtungen verschiedene Prüfmittel für den Flaschenkopf, für die Seitenwand und für den Boden. Sämtliche Prüfmittel sind für eine berührungslose Überprüfung ausgelegt, und zwar mit optischen Mitteln (Prospekt der Firma Holstein und Kappert, Dortmund: H&K-Inspektionsmaschine ALPHATRONIC S. 18 und 19). Von diesen bekannten Prüfmitteln lassen sich die für die Bodenkontrolle, die Mündungskontrolle und die Restflüssigkeitskontrolle vorgesehene Prüfmittel ohne weiteres bei Bügelverschlußflaschen einsetzen. Nicht ohne weiteres verwendbar sind aber die Prüfmittel für die Seitenwandkontrolle, weil störend im Strahlungsweg dieser Prüfmittel der am Flaschenhals anliegende Bügelverschluß liegt.

Störend bei der Seitenwandkontrolle sind auch Reliefs in der Flaschenwand. Deshalb wird bei einer bekannten Inspektionsmaschine darauf verzichtet, die Seitenwand in der vollen axialen Höhe zu kontrollieren. Man beschränkt sich auf die axialen Bereiche, die kein Relief tragen.

Der Erfindung liegt die Aufgabe zugrunde, eine Inspektionsmaschine für Bügelverschlußflaschen zu schaffen, die eine optimale Kontrolle des Verschlußstöpsels ermöglicht.

Diese Aufgabe wird bei einer Inspektionsmaschine der eingangs genannten-Art dadurch gelöst, daß die Hubelemente den Oberbügel und den Unterbügel anhebend untergreifen und daß im Bereich der Prüfmittel ein zwischen den Schenkeln des angehobenen Oberbügels am Verschlußstöpsel angreifendes, fingerartiges Verdreh- und Ausrichtelement einführbar ist, das den Verschlußstöpsel mit seiner Gummidichtung in eine nach außen gerichtete Position zum Prüfmittel bringt und in dieser Position hält.

Bei der erfindungsgemäßen Inspektionsmaschine ist eine sichere Kontrolle des Verschlußstöpsels gewährleistet, weil, unabhängig von Erschütterungen der Flasche in der Inspektionsmaschine, die Verschlußstöpsel in eine definierte Lage gebracht und gehalten werden. Die räumlich exakte Positionierung des Verschlußstöpsels ist aufgrund des Zusammenspiels des Ober- und Unterbügel untergreifenden Hubelementes und des von oben einführbaren fingerartigen Ausrichtelementes gewährleistet, weil das Ausrichtelement gegen die Kraftrichtung der unterstützenden Kraft des Hubelementes bewegt wird. Dies vermögen an sich bekannte Verdreh- und Ausrichtelemente an Vorrichtungen zum Aufsetzen eines Verschlußstöpsels auf eine Flasche nicht zu leisten, weil dabei die unterstützende Gegenkraft fehlt (DE-AS 11 16 561).

Das Halten des Verschlußstöpsels bei der Verschlußstöpselkontrolle in einer definierten Lage kann weiter dadurch verbessert werden, daß das Verdreh- und Ausrichtelement einen federbelasteten Niederhalter trägt, der beim Einführen des Verdreh- und Ausrichtelementes zwischen die Schenkel des Oberbügels auf den Oberbügel einwirkt, indem er ihn auf das Hub- und Stützelement drückt. Bei dieser Ausgestaltung der Erfindung wird mit einfachen Mitteln gewährleistet, daß der Oberbügel bei der Stöpselkontrolle auf dem Hub- und Stützelement aufliegt, indem der Gelenkpunkt des Verschlußstöpsels räumlich fixiert ist. Die für die fehlerfreie Kontrolle dann noch erforderliche Ausrichtung gewährleistet das Verdreh- und Ausrichtelement.

Ferner ist es für eine genaue räumliche Positionierung des Spannbügels nützlich, wenn jedem Hub- und Stützelement ein Spannelement zugeordnet ist, zwischen dem und dem Hub- und Stützelement der vom Hub- und Stützelement unterstützte Spannbügel, insbesondere an seinem Gelenk, einspannbar ist. Auch diese Maßnahme trägt zur räumlichen Fixierung des Bügelverschlusses bei der Kontrolle bei.

Nach einer Ausgestaltung der Erfindung kann das fingerartige Verdreh- und Ausrichtelement eine Profilierung aufweisen, die am Verschlußstöpsel mit Ausnahme seiner glatten Stirnseite beim Einführen verdrehend wirkt. Die genaue Positionierung des Verschlußstöpsels kann weiter dadurch verbessert werden, daß jedem Hubelement ein Spannelement zugeordnet ist, zwischen dem und dem Hubelement der vom Hubelement unterstützte Unterbügel einspannbar ist. Auf diese Art und Weise wird die Verschwenkachse des Oberbügels hinsichtlich seiner axialen und radialen Lage zur Flasche festgelegt.

Die für die genaue Positionierung des Verschlußstöpsels vorgesehenen Mittel lassen sich auch mit der Seitenwandkontrolle kombinieren. Sofern nämlich die Hubelemente ein in einem 90° Drehbereich wirksames Gleitstück für die Bügel bilden, können die Flaschen durch die drehgesteuerten Drehteller um 90° verdreht werden und die Seitenwandkontrolle kann auch im Halsbereich ohne Behinderung durch den Bügelverschluß vorgenommen werden.

Die im allgemeinen problematische Seitenwandkontrolle bei Flaschen mit Reliefs in der Seitenwand läßt sich bei der Erfindung problemlos durchführen, denn durch die Drehsteuerung der Drehteller können die Flaschen in eine vorbestimmte Drehstellung zu den Prüfmitteln für die Seitenwandkontrolle verdreht werden. Wenn nämlich zwischen den Reliefs in Umfangsrichtung Lücken sind, kann die Seitenwandkontrolle durch die Lücken erfolgen. Diese Art der Seitenwandkontrolle ist allerdings nicht auf Bügelverschlußflaschen beschränkt, sondern läßt sich immer dann verwirklichen, wenn die Flaschen auf drehgesteuerten Drehtellern angeordnet sind und Mittel zum Ausrichten der Drehstellung der Flaschen vorgesehen sind.

Es versteht sich, daß neben diesen Prüfmitteln für die Seitenwandkontrolle und den Verschlußstöpsel die anderen bekannten Prüfmittel für die Bodenkontrolle, die Mündungskontrolle und die Restflüssigkeitskontrolle in der Inspektionsmaschine in an sich bekannter Weise intregriert sein können, so daß eine vollständige Inspektion der Bügelverschlußflaschen erreicht wird.

Das für die Verschlußstöpselkontrolle eingesetzte Hub- und Stützelement läßt sich mit verhältnismäßig einfachen Mitteln auch bei der Seitenwandkontrolle der Flasche benutzen, bei der es darauf ankommt, daß der Verschlußstöpsel nicht seitlich an der bis einschließlich des Flaschenhalses zu kontrollierenden Flasche anliegt. Diese sogenannte Seitenwandkontrolle erfolgt beim Einsatz des Hub- und Stützelementes vorzugsweise in einer Drehstellung, in der sich der Verschlußstöpsel nicht in der für die Verschlußstöpselkontrolle günstigen Außenlage, sondern in einer dazu um 90° verdrehten Position zwischen den in den Aufnahmeplätzen gehaltenen Flaschen befindet, weil dann der vom Oberbügel herabhängende Stöpsel nicht die Durchleuchtung der Flasche im wesentlichen in radialer Richtung des Drehtisches behindert. Um auch bei enger Anordnung der Flaschen diese Verdrehung zu ermöglichen, ist nach einer Ausgestaltung der Erfindung vorgesehen, daß die Hub- und Stützelemente gabelförmig ausgebildet sind und für den Verschlußbügel ein im Drehbereich der Flasche wirksames Gleitstück und an einem Gabelast eine rampenartige, nur den Oberbügel untergreifende Steigkurve bilden, wobei in dem Umfangsbereich des Drehtisches, in dem nach Verdrehung der Flasche das Gleitstück und die Steigkurve wirksam sind, optische Prüfmittel für die Seitenwandkontrolle der Flasche vorgesehen sind. Mit dieser Ausgestaltung der Erfindung wird erreicht, daß bei enger Anordnung der Flaschen es nicht zu einer Kollision der Verschlußstöpsel benachbarter Flaschen kommt, wenn diese Flaschen für die Seitenwandkontrolle in verschiedene Drehstellungen nacheinander gebracht werden, um die Wand auf ihrem gesamten Umfang zu durchleuchten.

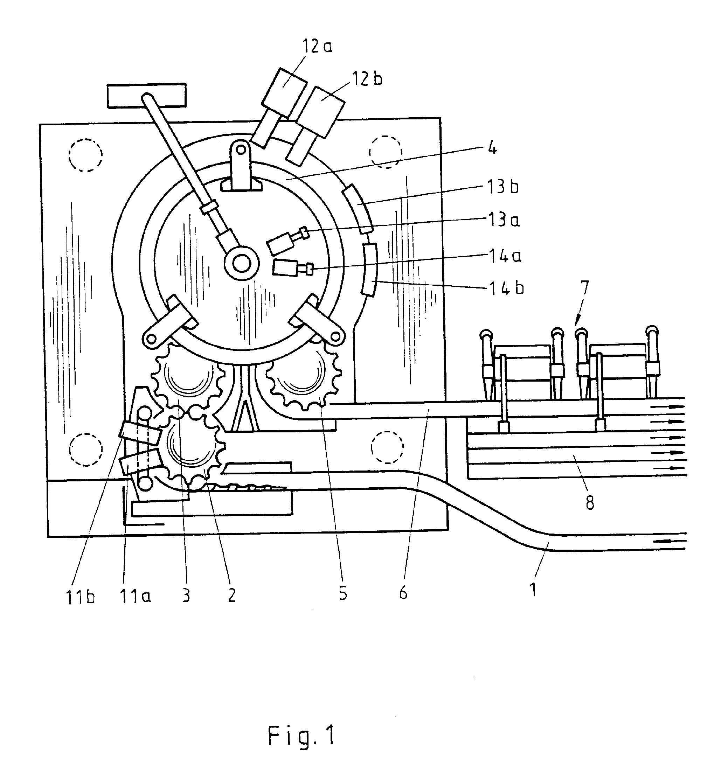

Im folgenden wird die Erfindung anhand einer drei Ausführungsbeispiele darstellenden Zeichnung näher erläutert. Im einzelnen zeigen
- Fig. 1: eine Inspektionsmaschine für Bügelverschlußflaschen in schematischer Darstellung,
- Fig.2a: einen Drehtisch der Inspektionsmaschine gemäß Fig.1 in einer ersten Ausführung im halben Axialschnitt,
- Fig.2b: einen Drehtisch der Inspektionsmaschine gemäß Fig.1 in einer zweiten Ausführung im halben Axialschnitt,
- Fig. 3: den Drehtisch der Inspektionsmaschine gemäß Fig. 1 ausschnittweise in einem zu Fig. 2a,2b um 90° verdrehten Axialschnitt,
- Fig. 4 und 5: den Drehtisch der Inspektionsmaschine gemäß Fig. 1 im Axialschnitt in verschiedenen Phasen der Ausrichtung einer Bügelverschlußflasche,

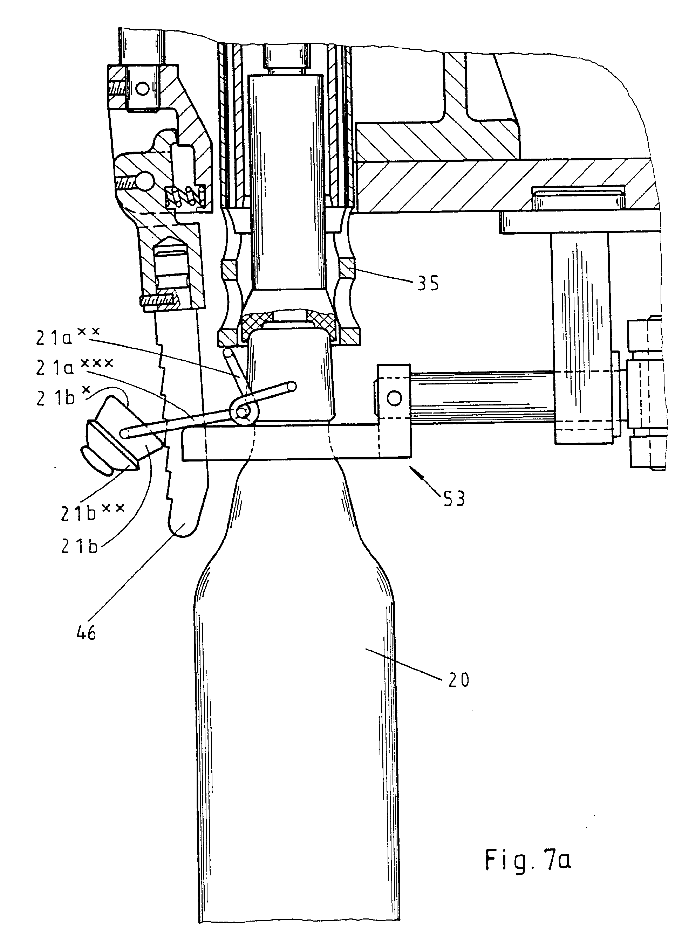

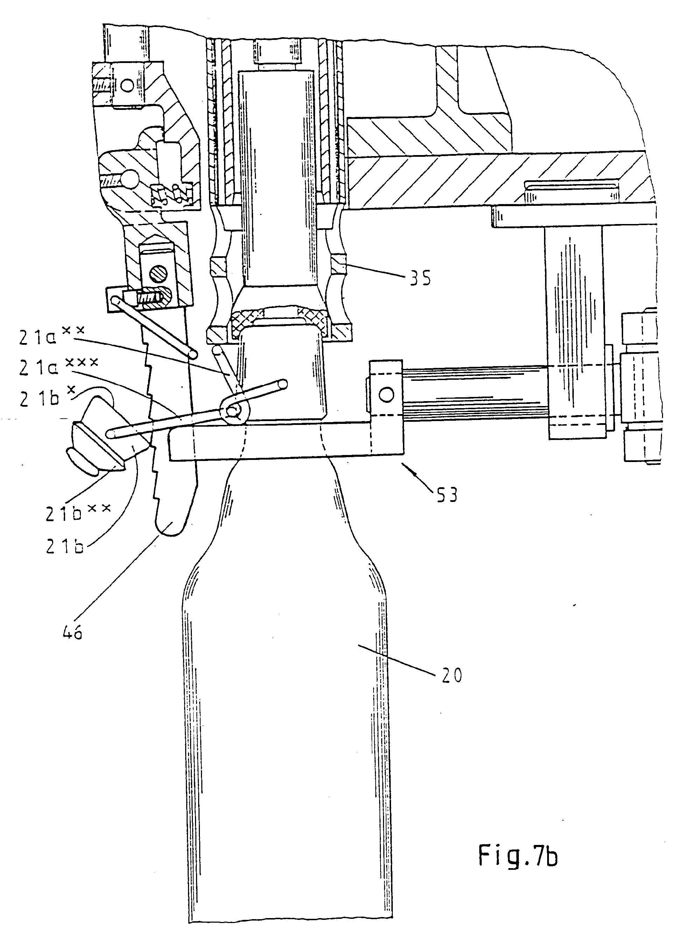

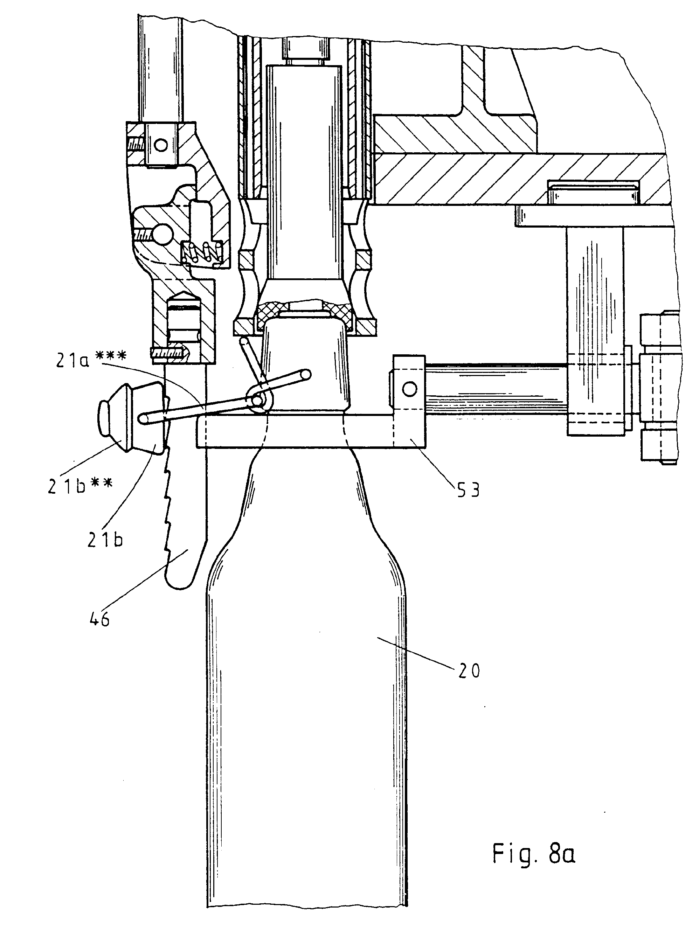

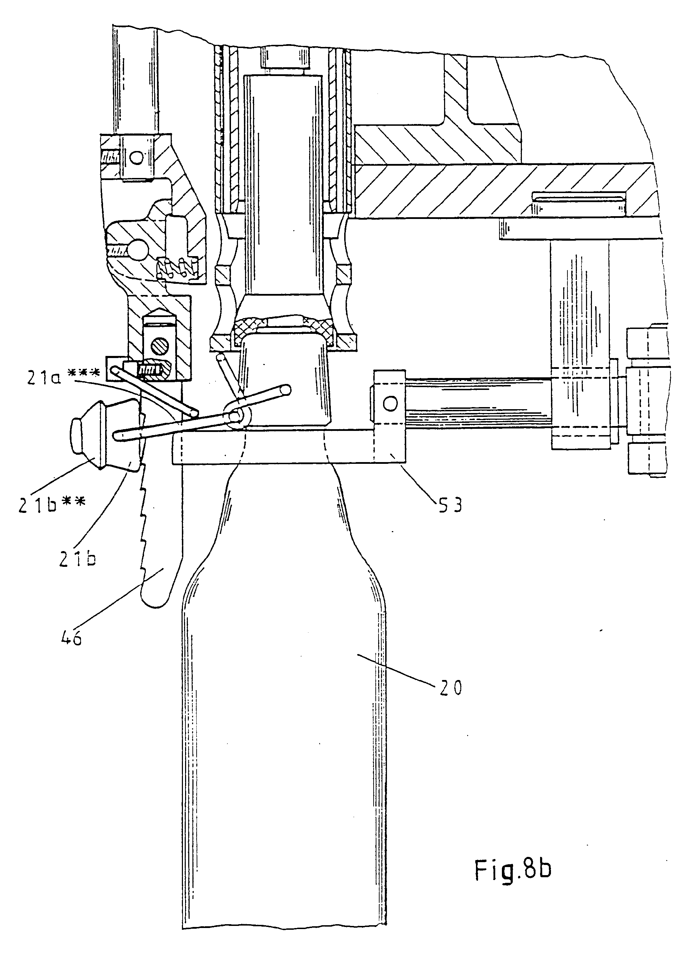

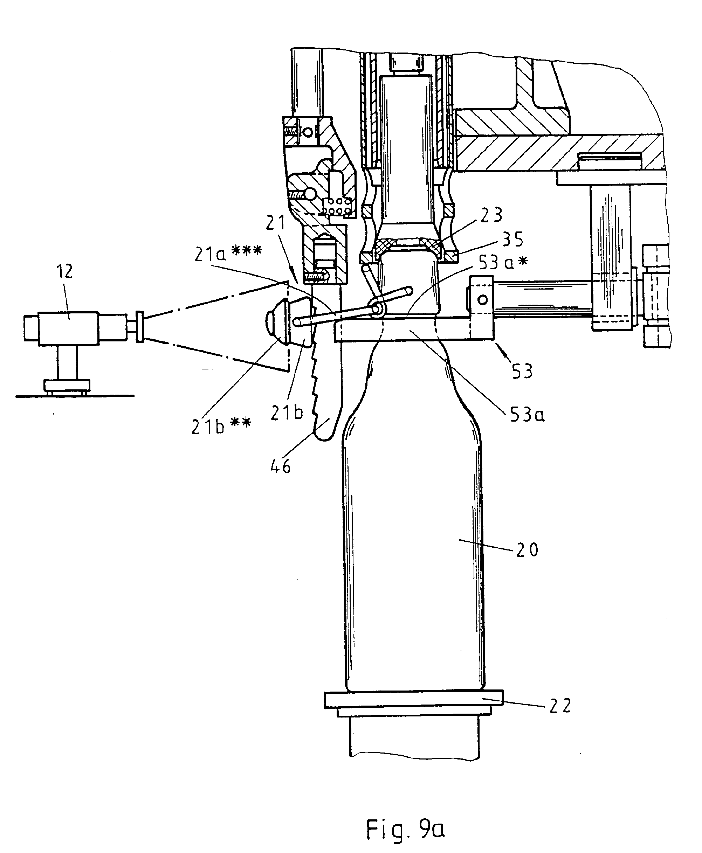

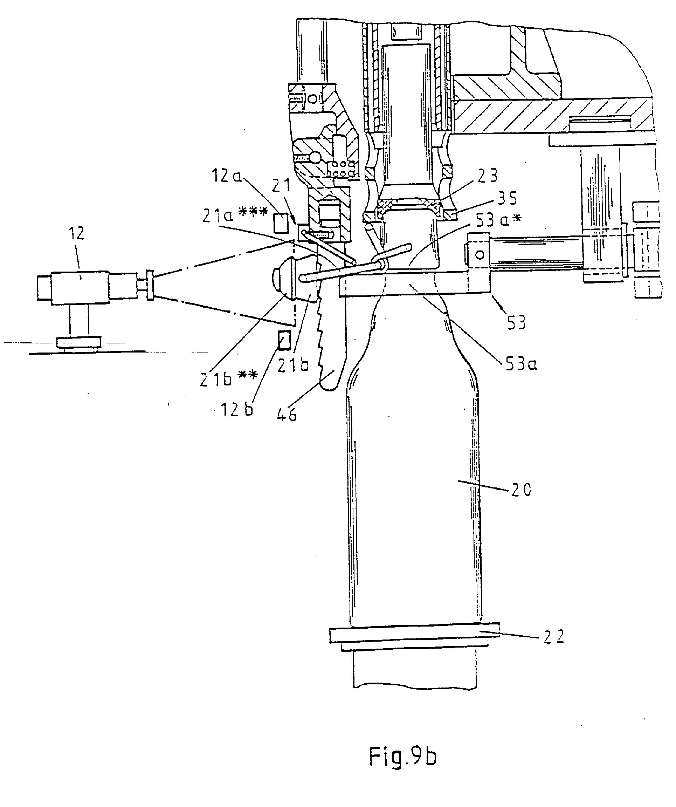

- Fig. 6a,6b bis 9a,9b: den Drehtisch der Inspektionsmaschine gemäß Fig. 1 in den beiden Ausführungen ausschnittweise im Axialschnitt in verschiedenen Phasen der Verschlußstöpselkontrolle,
- Fig. 10a,10b: den Drehtisch der Inspektionsmaschine gemäß Fig. 1 in der ersten und dritten Ausführung ausschnittweise im Axialschnitt bei der Seitenwandkontrolle,
- Fig. 11: den Drehtisch gemäß Fig. 1 ausschnittweise im Querschnitt nach Linie I-I der Fig. 10a,
- Fig. 12: den Drehtisch gemäß Fig. 1 ausschnittweise im Axialschnitt in einer dritten Ausführung der Fig. 10b in der Phase der Verschlußstöpselkontrolle,
- Fig. 13: den Drehtisch gemäß Fig. 1 ausschnittweise im Querschnitt nach Linie II-II der Fig. 12,
- Fig. 14: den Drehtisch gemäß Fig. 1 in der dritten Ausführung der Fig. 10b ausschnittweise im Axialschnitt in der Phase der Verschlußstöpselkontrolle,
- Fig. 15: zwei benachbarte Hub- und Stützelemente des Drehtisches gemäß Fig. 1 in der dritten Ausführung der Fig. 10b in Aufsicht, und
- Fig. 16: die Dreh- und Hubelemente der Fig. 15 in Ansicht aus Richtung des Pfeiles A der Fig. 15.

Über einen als Förderband ausgebildeten Förderer 1 werden Bügelverschlußflaschen in Reihe einem ersten Einlaufstern 2 zugefördert. Von diesem ersten Einlaufstern 2 gelangen die Flaschen über einen zweiten Einlaufstern 3 zu einem Drehtisch 4, der am Umfang für die Flaschen Aufnahmeplätze hat. In dem Drehtisch 4 werden die Flaschen bezüglich ihrer Bügelverschlüsse ausgerichtet und inspiziert. Vom Drehtisch 4 gelangen die Flaschen über einen Auslaufstern 5 auf einen einsträngigen Förderer 6, der über eine Schleuse 7 in einen mehrsträngigen Förderer 8 übergeht. Durch die Schleuse 7 werden die als nicht einwandfrei befundenen Bügelverschlußflaschen von dem durchgängigen Förderer 6 auf einen der Förderer des mehrsträngigen Förderers 8 übergeben.

Die Inspektionsmaschine weist eine mehrteilige optische Prüfeinrichtung auf. Im Bereich des ersten Einlaufsterns 2 sind doppelt installierte Prüfmittel 11a, 11b für die Mündungskontrolle, für die Restflüssigkeitskontrolle und für die Bodenkontrolle angeordnet, während im Bereich des Drehtisches 4 ebenfalls doppelt installierte Prüfmittel 12a, 12b, 13a, 13b für den Verschlußstöpsel und für die Seitenwandkontrolle vorgesehen sind.

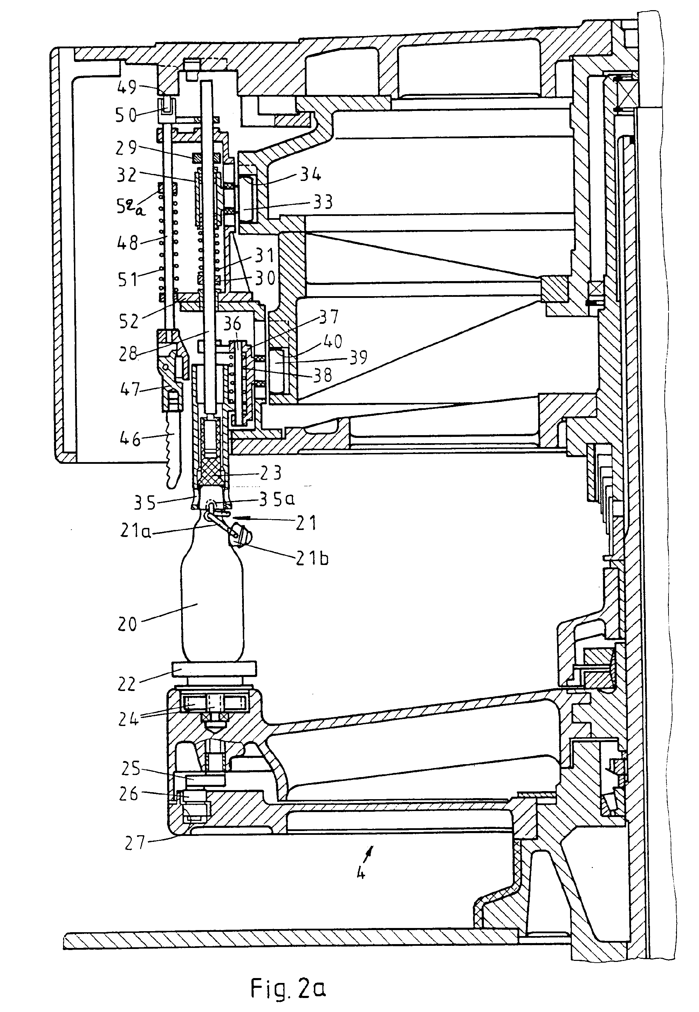

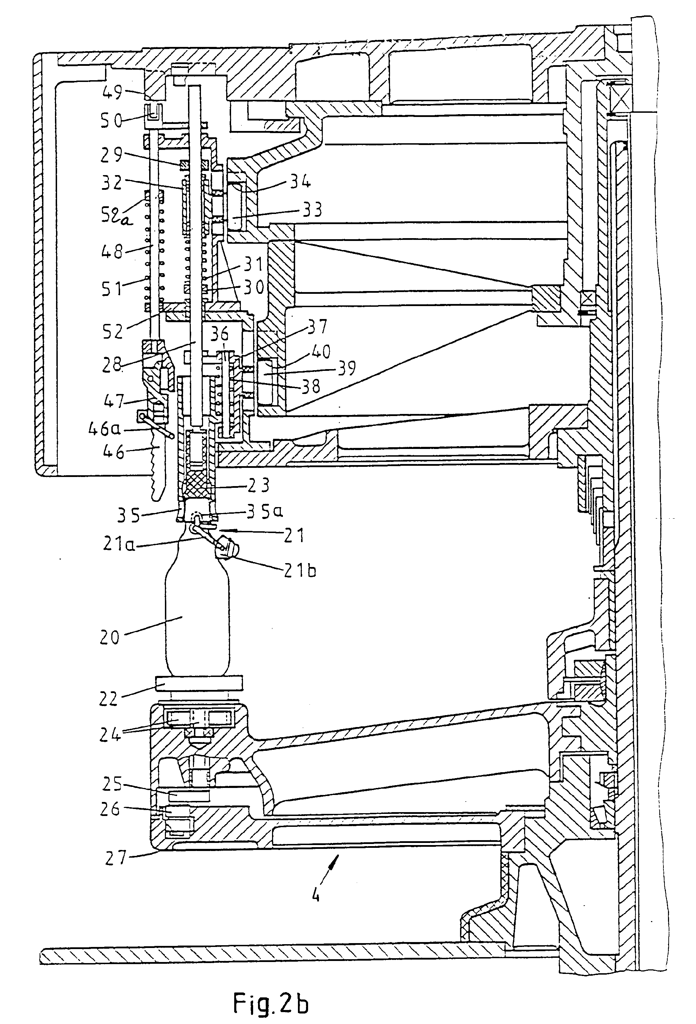

Im einzelnen ist der Aufbau und die Funktion der Inspektionsmaschine wie folgt: Die mit jeweils aus einem Verschlußbügel 21a und einem Verschlußstöpsel 21b bestehenden Bügelverschlüsse 21 zu verschließenden Bügelverschlußflaschen gelangen in zufälliger Drehstellung auf die um Umfang des Drehtisches 4 angeordneten Aufnahmeplätze. Wie die Fig. 2a,2b zeigen, besteht jeder Aufnahmeplatz aus einem Drehteller 22 und einem Zentrierkopf 23. Zwischen dem Drehteller 22 und dem Zentrierkopf 23 ist die Flasche 20 axial einspannbar. Jedem Drehteller 21 ist ein kurvengesteuertes Getriebe zugeordnet, das aus einem Zahnradgetriebe 24, einer Kurbel 25 und einem Eingriffsglied 26 und einer ortsfesten, ebenen Nutkurve 27 besteht. Bei Drehung des Drehtisches 4 wird in Abhängigkeit von dem Verlauf der Nutkurve 27 die Kurbel 25 verschwenkt und damit der Drehteller 20 verdreht.

Der Zentrierkopf 23 wird von einer axial verschiebbaren Stange 28 getragen, auf der zwischen einem festen Anschlag 29 auf der Stange 28 und einer an einem ebenfalls auf der Stange 28 festen Widerlager 30 abgestützten Feder 31 eine Büchse 32 verschiebbar sitzt. Die Lage der Büchse 32 wird von einem von ihr getragenen Eingriffsglied 33 bestimmt, das in einer als Nutkurve ausgebildeten gestellfesten Zylinderkurve 34 geführt ist. Der Zentrierkopf 23 ist wegen der Feder 31 nachgiebig an der Büchse 32 abgestützt.

Der Zentrierkopf 32 ist von einer Glocke 35 umgeben, die an ihrem unteren Rand zwei diametral gegenüberliegende, axiale Führungsschlitze 35a zur Aufnahme von Teilen des Verschlußbügels 21a der Flasche 20 hat. Die Glocke 35 ist auf Führungsstangen 36 eines Halters 37 gegen die Kraft von Federn 38 auf den Führungsstangen 36 von der Flasche 20 wegverschiebbar. Der Kalter 37 trägt ein Eingriffsglied 39, das in einer als Nutkurve ausgebildeten gestellfesten Zylinderkurve 40 eingreift.

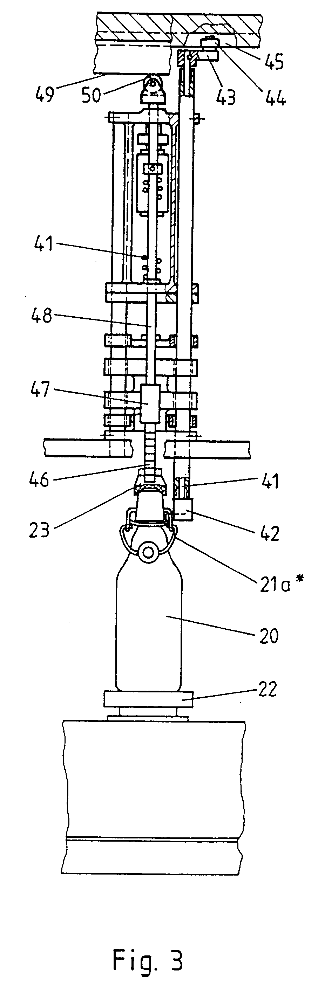

Wie die Figuren 3,4,5 zeigen, ist seitlich neben einem jeden Flaschenkopf ein mittels einer Steuerstange 41 verschwenkbarer Stößel 42 angeordnet. Die Steuerstange 41 weist an ihrem dem Stößel 42 gegenüberliegenden Ende einen Schwenkarm 43 mit einem Eingriffsglied 44 auf, das in eine gestellfeste, ebene Nutkurve 35 eingreift. Der Stößel 42 wirkt auf einen Schenkel des Unterbügels 21a* des Verschlußbügels 21a ein.

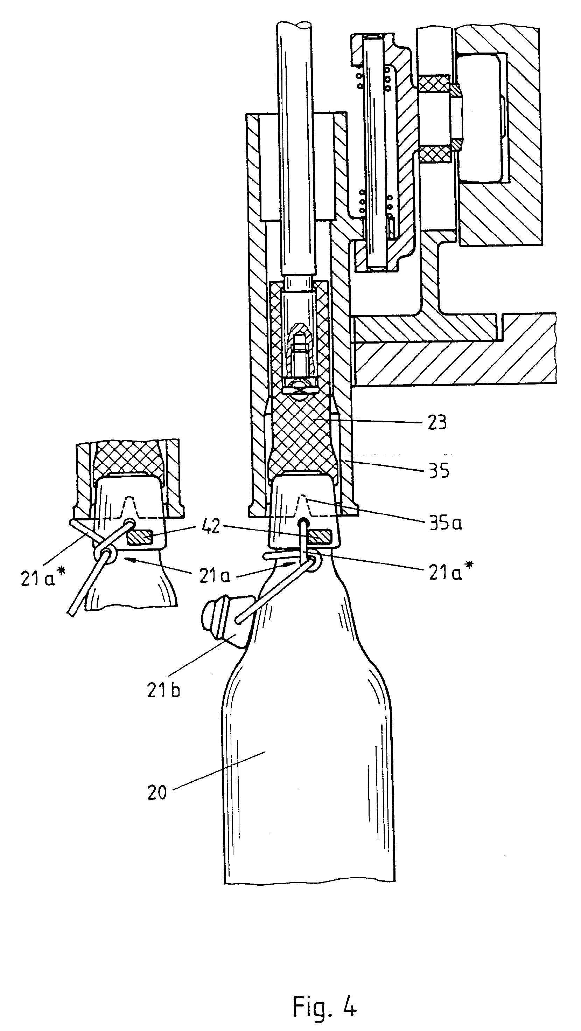

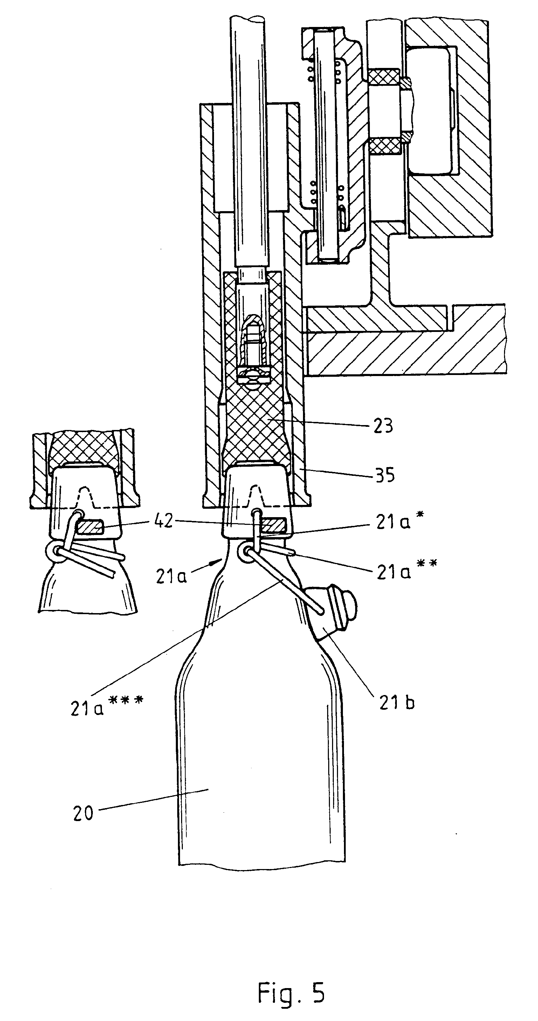

Das Ausrichten der Flaschen 20 bezüglich der Drehstellung des Bügelverschlusses 21 erfolgt bei axial eingespannter Flasche. Nachdem die Flasche 20 in dem Aufnahmeplatz des Drehtisches 4 gelangt ist, wird der Zentrierkopf 23 in Abhängigkeit vom Verlauf der Steuerkurve 34 auf die Flasche 20 abgesenkt und die Flasche 20 axial eingespannt. Dabei wird die axiale Einspannkraft durch die Feder 31 begrenzt. Beim weiteren Transport wird die Steuerkurve 40 wirksam und senkt die Glocke 35 ab. Sofern die Flasche 20 sich dann nicht zufällig in einer von zwei möglichen Drehstellungen befindet, in der die Schenkel des Unterbügels 21a* des Verschlußbügels 21a mit den Führungsschlitzen 35a fluchten, setzt sich der untere Rand der Glocke 35 auf die Schenkel. Im Anschluß daran bewirkt die Kurve 27 eine mindestens 180°-Drehung des Drehtellers 22. Die Flasche 20 macht diese Drehbewegung mit, bis daß die Schenkel des Unterbügels 21a* in die Führungsschlitze 35a der durch die Feder 38 vorgespannten Glocke 35 einrasten. Von diesem Augenblick an führt der Drehteller 22 seine restliche Drehbewegung unter Schlupf gegenüber der Flasche 20 aus. Nach Beendigung dieser Drehbewegung der Drehteller 22 wird die Steuerung durch die Kurve 40 wirksam und die Glocke 35 wieder angehoben. Nach Freigabe der Schenkel des Unterbügels 21a* wird durch die Steuerung der Kurve 45 der Stößel 42 gegen eine der beiden seitlichen Schenkel des Unterbügels 21a* bewegt (Figur 4 und 5). Sofern sich die Flasche 20 in der in Figur 4 gezeigten Position befindet, weicht der Unterbügel 21a* aus. Sofern sich die Flasche 20 aber in der dazu 180° versetzten Position der Figur 5 befindet, kann der Unterbügel 21a* nicht ausweichen, weil er daran durch sein zur Anlage an der Flasche 20 gekommenes Spannglied 21a** gehindert wird. Infolge dessen bewirkt der Stößel 42 eine Verdrehung der Flasche 20 soweit, bis daß beim anschließenden erneuten Absenken der Glocke 35 die Schenkel des Unterbügels 21a* nicht in die Führungsschlitze 35a einrasten können.

Anschließend erfolgt eine weitere Drehung des Drehtellers 22 um mindestens 180°. Sofern die Flasche durch den Stößel 32 nicht verdreht wurde, so daß beim erneuten Absenken der Glocke die Schenkel 21a* wieder in die Führungsschlitze 35a einrasten konnten, wird die Flasche 20 von dem Drehteller 22 nicht mitgenommen. Im anderen Fall dagegen macht die Flasche 20 die Drehbewegung mit, bis daß die Schenkel des Unterbügels 21a* in die Führungsschlitze 35a der Glocke 35 einrasten. Durch diese wiederholte Drehbewegung des Drehtellers 22 wird erreicht, daß in jedem Fall die Flasche 20 in die gewünschte ausgerichtete Position mit zur von der Mitte des Drehtisches 4 wegweisendem Bügelverschluß 21 gedreht wird.

Auf der bezogen auf die Mitte des Drehtisches 4 liegenden Außenseite der Flasche 20 ist ein fingerartiges Verdreh- und Ausrichtelement 46 mit einer stirnseitigen zahnartigen Profilierung 46a angeordnet, die über einen Halter 47 von einer Steuerstange 48 getragen ist. Der Halter 47 ist als Kniegelenk ausgebildet und durch eine Feder 47a vorgespannt, so daß bei Druck auf die stirnseitige Verzahnung das Dreh- und Ausrichtelement 46 ausweicht. Die Steuerstange 48 trägt an ihrem dem Verdreh- und Ausrichtelement 46 gegenüberliegenden Ende ein an einer ortsfesten Steuerkurve 49 abgestütztes Eingriffsglied 50. Mit einer Feder 51, die zwischen einem gestellfesten Widerlager 52 und einem auf der Steuerstange 48 fest angeordneten Widerlager 52a angeordnet ist, wird das Eingriffsglied 50 der Steuerstange 48 in Anlage an der Kurve 49 gehalten.

Von dem ersten Ausführungsbeispiel der mit dem zusätzlichen Buchstaben "a" bezeichneten Figuren unterscheidet sich das zweite Ausführungsbeispiel in den mit dem zusätzlichen Buchstaben "b" bezeichneten Figuren darin, daß der Kalter 47 des Verdreh- und Ausrichtelementes 46 einen bügelartigen, als Feder ausgebildeten Niederhalter 46b trägt, der auf der der Drehtischmitte zugeordneten Seite des Verdreh- und Ausrichtelementes 46 angeordnet ist.

In beiden Ausführungsbeispielen ist jedem Aufnahmeplatz ferner ein am Verschlußbügel 21a angreifendes Hubelement 53 zugeordnet, das als Gabel 53a mit zwei Gabelästen ausgebildet ist und dessen Oberseite ein Gleitstück 53a* für den Bügelverschluß 21a bildet. Das Hubelement 53 wird von einer radial aussteuerbaren Steuerstange 54 getragen, die über ein Kniegelenk 55 und eine Welle 56 mit einer Kurbel 57 verbunden ist, die sich mit einem Eingriffsglied 58 an einer gestellfesten, ebenen einseitigen Steuerkurve 59 abstützt. Mittels einer Feder 60 wird das Eingriffsglied 58 nachgiebig gegen die Kurve 59 gedrückt.

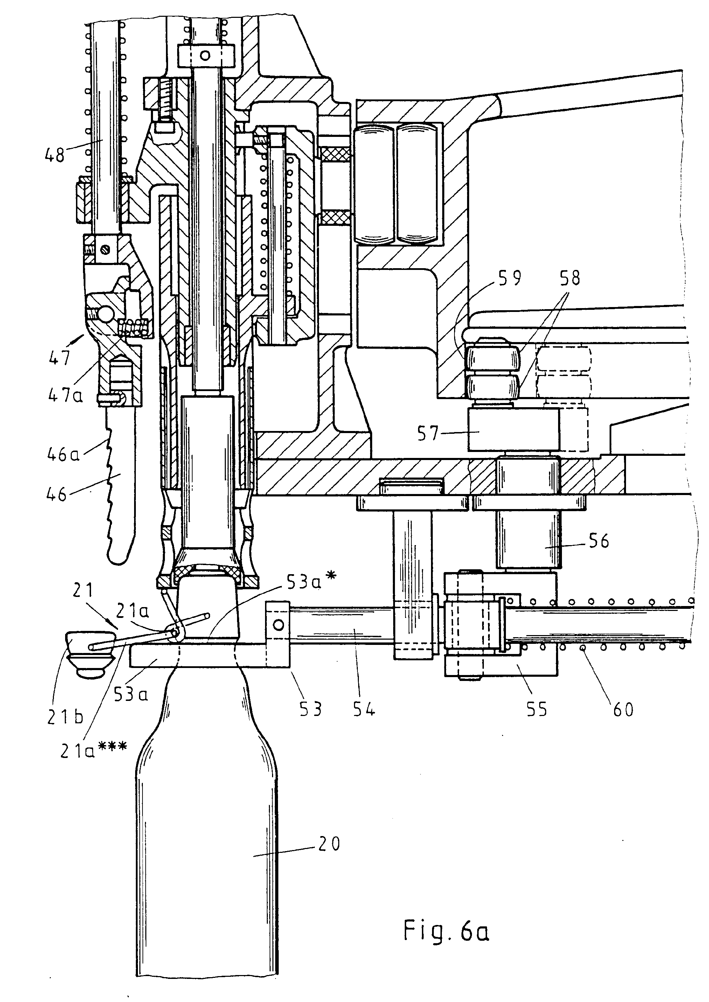

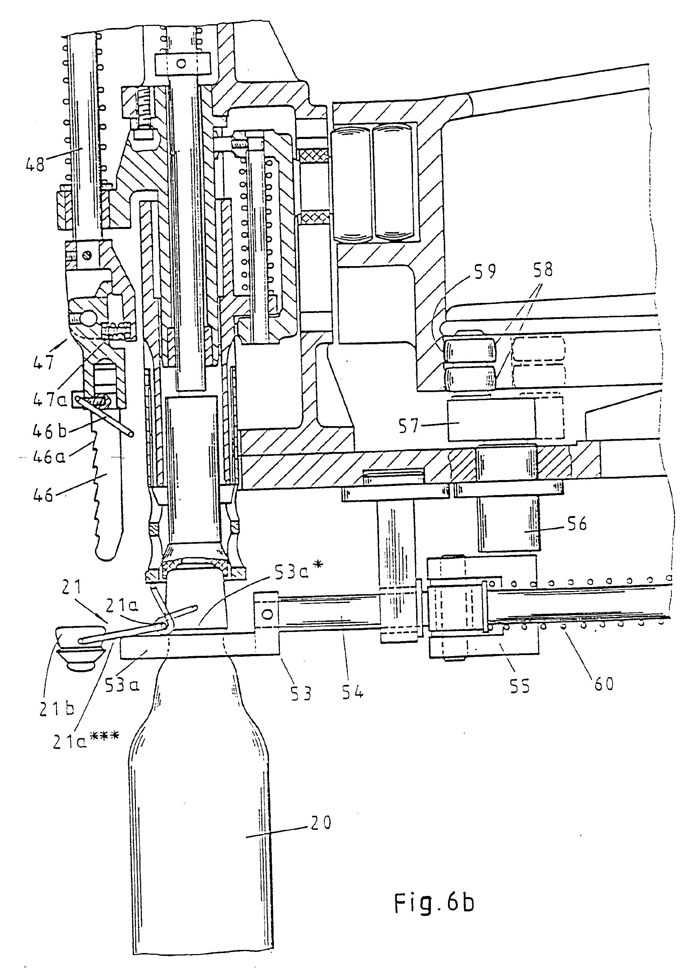

Nachdem die Flasche 20, wie beschrieben, in ihrer Drehstellung derart ausgerichtet ist, daß der Bügelverschluß 21 bezogen auf die Mitte des Rundläufers 4 außen liegt, wird das Hubelement 53 aus seiner zurückgezogenen, außerhalb der Flasche 20 liegenden Stellung in die in Fig. 6 dargestellte Position vorgesteuert. Dabei untergreifen seine Gabeläste 53a den Verschlußbügel 21a und heben ihn soweit an, bis daß Unter- und Oberbügel 21a*, 21a*** etwa fluchten. Anschließend wird das fingerartige Verdreh- und Ausrichtelement 46 nach unten bewegt, und zwar zwischen den Schenkeln des Oberbügels 21a***. Dabei greift es mit seiner verzahnten Vorderkante 6a am Verschlußstöpsel 21b an und verdreht ihn soweit, bis daß seine glatte Stirnseite 21b* an dem Verdreh- und Ausrichtelement 46 anliegt. Bei diesem Verdreh- und Ausrichtvorgang sorgt die nachgiebige Abstützung des Ausrichtelementes 46 durch die Feder 47a dafür, daß das Verdreh- und Ausrichtelement 46 zwar ausweichen kann, gleichwohl aber sicher am Verschlußstöpsel 21a in Anlage gehalten wird. An der glatten Stirnseite 21b* findet das Verdreh- und Ausrichtelement 46 keinen weiteren Angriffspunkt, so daß es bei der weiteren Bewegung darüber hinweggleitet und den Verschlußstöpsel 21b mit nach außen gerichteter Dichtung 21b** fixiert hält.

In der unteren Position des Verdreh- und Ausrichtelementes 46 drückt der vom Halter 47 getragene, bügelartige und als Feder ausgebildete Niederhalter 46 auf die Schenkel des Oberbügels 21a*** derart, daß der Oberbügel 21a*** an den Gabelästen 53a des Hub- und Stützelementes 53 anliegt.

Bei diesem Verdreh- und Ausrichtvorgang kann hilfsreich sein, wenn die Glocke 35 mit ihrem stirnseitigen Rand den Spannbügel 21a** gegenüber den Gabelästen 53a axial einspannt. In dieser ausgerichteten Position passiert der Verschlußstöpsel 21b das Prüfmittel 12a, 12b, das als Kamera ausgebildet sein kann und den Verschlußstöpsel 21b bezüglich der Dichtung 21b** überprüft.

Nach Überprüfung des Verschlußstöpsels 21b wird das Verdreh- und Ausrichtelement 46 zurückgezogen und die Glocke 35 angehoben, so daß der Verschlußbügel 21a drucklos auf dem Gleitstück 53a* der Gabeläste 53a des Hub- und Stützelementes 53 liegt.

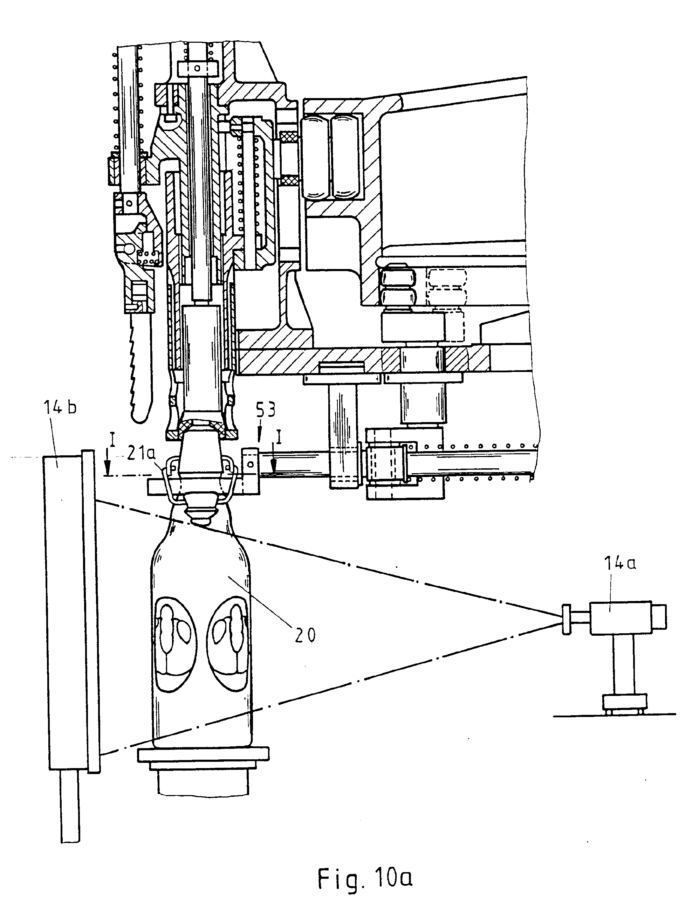

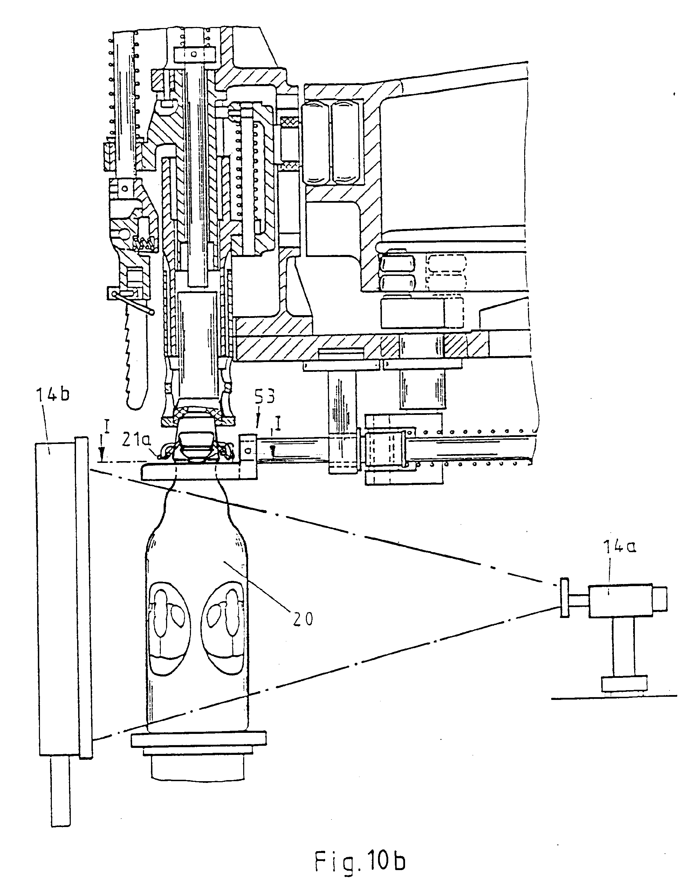

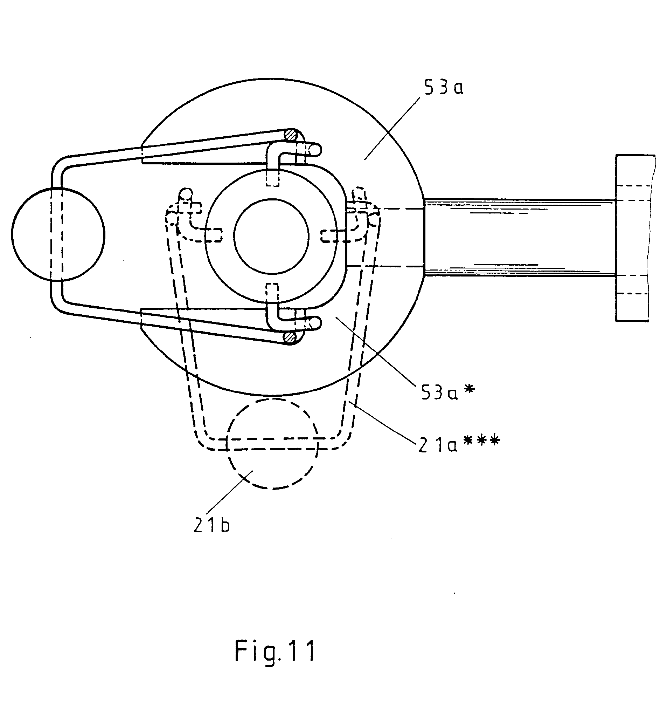

Im Bereich eines dritten Förderabschnittes des Drehtisches 4, in dem die Seitenwandkontrolle stattfindet, erfolgt eine Verdrehung der Flasche 20, wobei der Verschlußstöpsel 21b auch in eine seitliche Position neben der Flasche 20 gebracht wird, wie in Fig. 10a, 10b und 11 dargestellt ist.

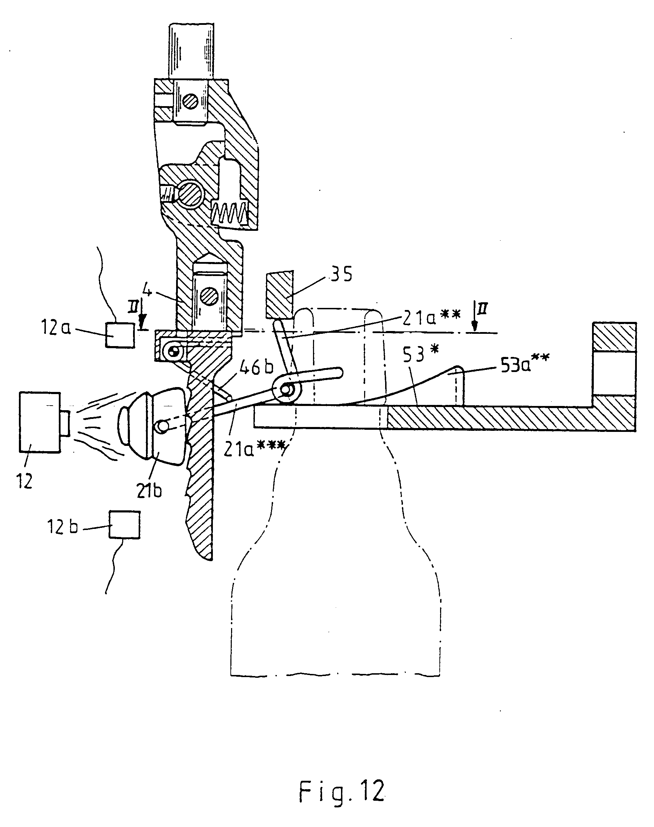

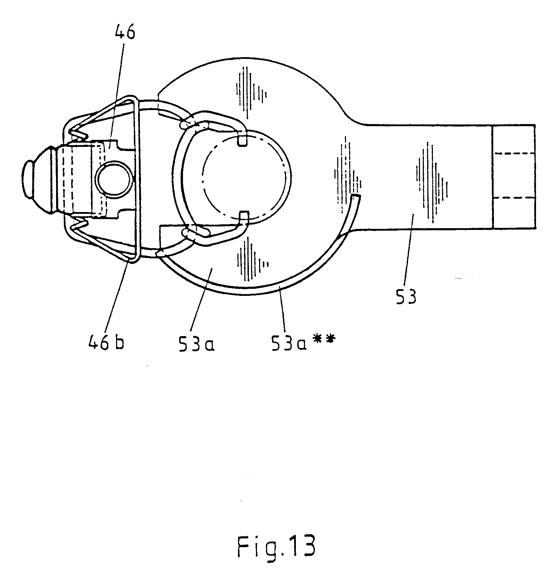

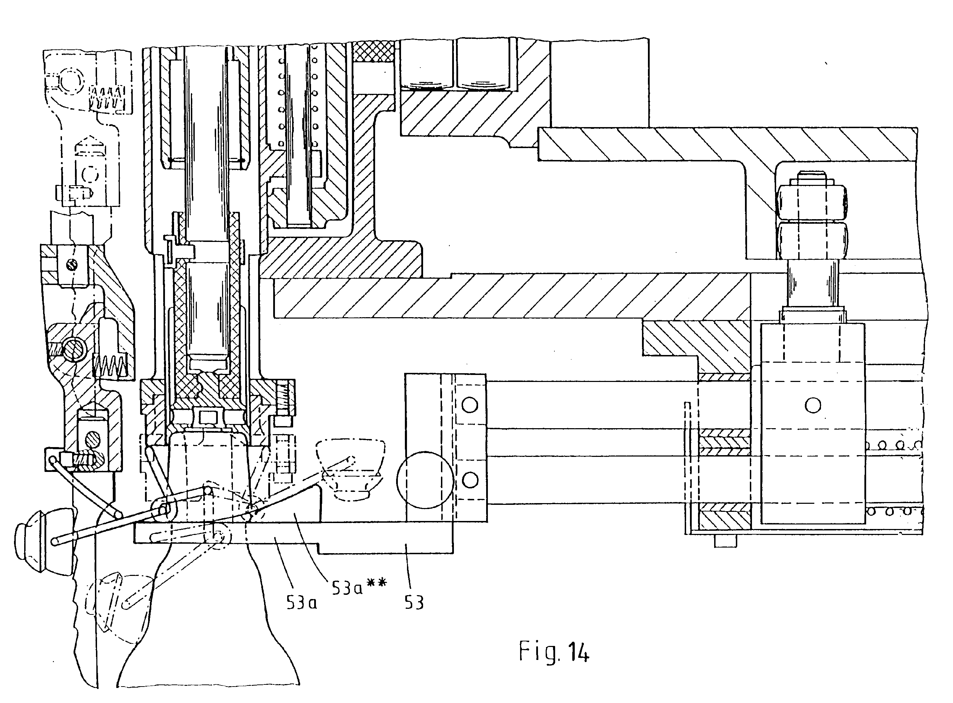

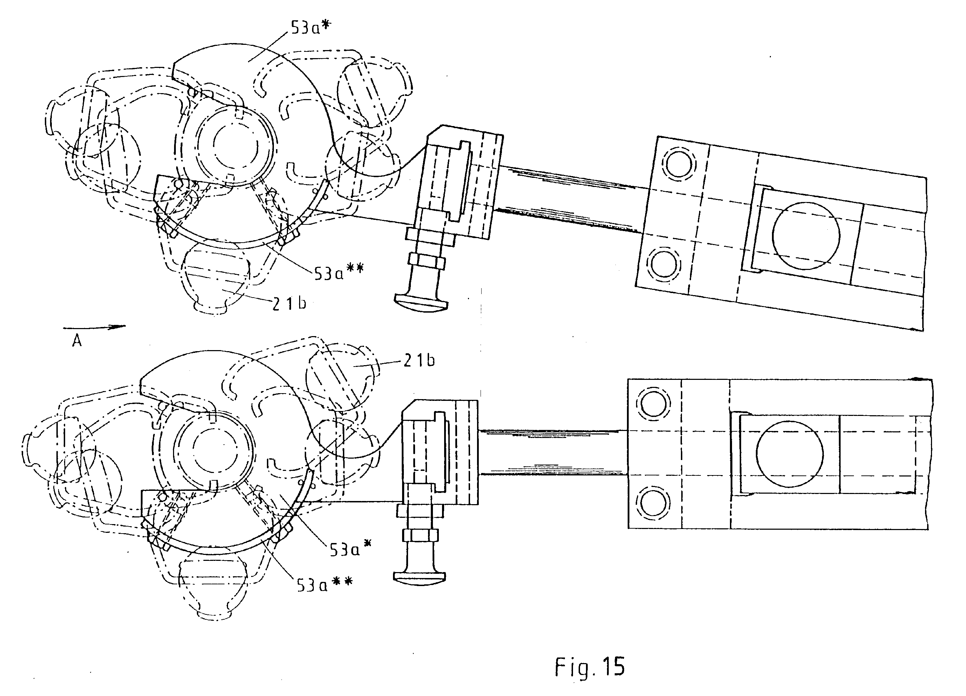

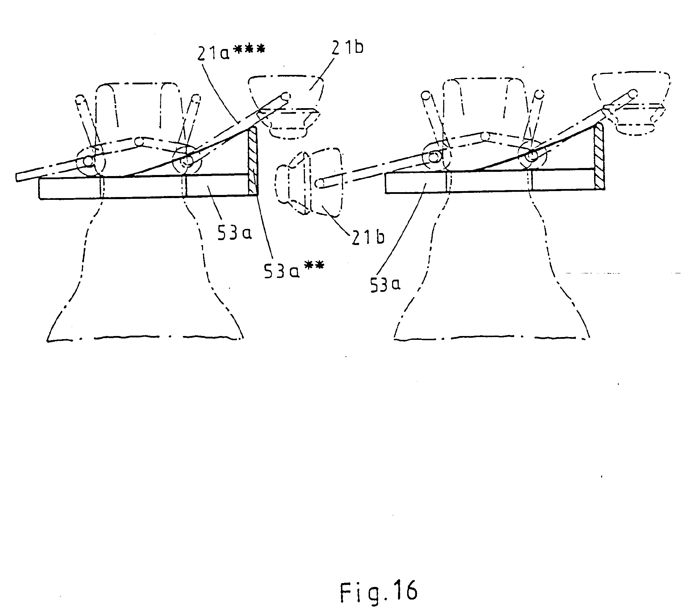

Je nach Art der Ausführung des Hubelementes 53 bleibt bei dieser Verdrehung der Oberbügel in der Ebene der ausgerichteten Position oder wird angehoben. Während der Ausführung gemäß Fig. 10a bleibt der Oberbügel 21*** in seiner Ebene, während er bei der dritten Ausführung gemäß den Figuren 10b, 12, 13, 14, 15 und 16 von einer von der Gabel 53a getragenen rampenartigen Steigkurve 53a** unterstützt und angehoben wird, so daß bei Verdrehung benachbarter Flaschen die Stöpsel 21b einander passieren können, wie in Figur 11 dargestellt ist. Dies ist vor allem dann wichtig, wenn die Aufnahmeplätze eng benachbart sind. Die Flasche 20 passiert dann den Strahlengang des Prüfmittels, das aus einer im Bereich des Drehtisches 4 angeordneten Kamera 14a und einer Lichtquelle 14b besteht. Dabei wird die Flasche 20 bei durch das Hubelement 53 angehobenem Verschlußbügel 21a von der im Bereich des Drehtisches 4 angeordneten, nach außen gerichteten Lichtquelle 14b durchstrahlt und durch die Kamera 14a in verschiedenen Drehstellungen überprüft.
In Abhängigkeit von den Inspektionsergebnissen werden die fehlerfreien Flaschen von den fehlerbehafteten Flaschen am Ausgang des Drehtisches in bekannter Weise über Schleusen auf verschiedene Förderstrecken gegeben.

## Patentansprüche

1. Inspektionsmaschine für Bügelverschlußflaschen, bestehend aus einem Drehtisch (4) mit an seinem Umfang angeordneten Aufnahmeplätzen, in denen die Flaschen (20) zwischen einem drehgesteuerten Drehteller (22) und einem auf die Flaschenmündung absenkbaren Zentrierkopf (23) axial einspannbar und mittels Ausrichtelementen (35,42) in eine Drehstellung mit nach außen liegendem Verschlußstöpsel (21b) verdrehbar sind, sowie mit an die Bügel (21a) der Bügelverschlüsse (21) ansetzbaren Hub- und Stützelementen (53), mit denen im Bereich einer am Weg der in den Aufnahmeplätzen gehaltenen Flaschen (20) ortsfest angeordneten, optischen Prüfeinrichtung (11a - 14b) die Verschlußstöpsel (21b) der Bügelverschlüsse (21) aus ihrer Lage am Flaschenhals in eine angehobene, freihängende Lage überführbar sind, in der sie durch Prüfmittel (12a,12b) der Prüfeinrichtung (11a-14b) überprüfbar sind, **dadurch gekennzeichnet,** daß die Hubelemente (53) den Oberbügel (21a***) und den Unterbügel (21a*) anhebend untergreifen und daß im Bereich der Prüfmittel (12a,12b) ein zwischen den Schenkeln des angehobenen Oberbügels (21***) einführbares am Verschlußstöpsel (21b) angreifendes, fingerartiges Verdreh- und Ausrichtelement (46) angeordnet ist, das den Verschlußstöpsel (21) mit seiner Gummidichtung (21b**) in eine nach außen gerichtete Position zum Prüfmittel (12a,12b) bringt und in dieser Position hält.

2. Inspektionsmaschine nach Anspruch 1, **dadurch gekennzeichnet,** daß das Verdreh- und Ausrichtelement (46) einen federbelasteten Niederhalter (46a) trägt, der beim Einführen des Verdreh- und Ausrichtelementes (46) zwischen die Schenkel des Oberbügels (21a***) auf den Oberbügel (21a***) einwirkt, indem er ihn auf das Hub- und Stützelement (53) drückt.

3. Bügelflascheninspektionsmaschine nach Anspruch 1, oder 2, **dadurch gekennzeichnet,** daß jedem Hub- und Stützelement (53) ein Spannelement (35) zugeordnet ist, zwischen dem und dem Hub- und Stützelement (53) der vom Hub- und Stützelement (53) unterstützte Spannbügel (21a***), insbesondere an seinem Gelenk, einspannbar ist.

4. Inspektionsmaschine nach einem der Ansprüche 1 bis 3, **dadurch gekennzeichnet,** daß das fingerartige Verdreh- und Ausrichtelement (46) eine Profilierung (46a) aufweist, die am Verschlußstöpsel (21b) mit Ausnahme seiner glatten Stirnseite (21b*) beim Einführen verdrehend wirkt.

5. Inspektionsmaschine nach einem der Ansprüche 1 bis 4, **dadurch gekennzeichnet,** daß jedem Hubelement (53) ein Spannelement (35) zugeordnet ist, zwischen dem und dem Hubelement (43) der vom Hubelement (53) unterstützte Spannbügel (21a**) einspannbar ist.

6. Inspektionsmaschine nach einem der Ansprüche 1 bis 5, **dadurch gekennzeichnet,** daß das Verdreh- und Ausrichtelement (46) an einer Feder (47a) nachgiebig abgestützt ist.

7. Inspektionsmaschine nach einem der Ansprüche 1 bis 6, **dadurch gekennzeichnet,** daß die Hubelemente ein in einem 90° Drehbereich wirksames Gleitstück (53a*) für die Bügel (21a*,21a**) bilden, wobei bei wirksamem Gleitstück (53a*) die Flaschen (20) durch die drehgesteuerten Drehteller (22) um 90° verdreht werden, und daß die optische Prüfeinrichtung Prüfmittel (13a - 14b) für die Seitenwandkontrolle aufweist.

8. Inspektionsmaschine nach Anspruch 7, **dadurch gekennzeichnet,** daß die drehgesteuerten Drehteller (22) die Flaschen (20) in eine vorbestimmte Drehstellung zu den Prüfmitteln (13a - 14b) für die Seitenwandkontrolle verdrehen.

9. Inspektionsmaschine nach einem der Ansprüche 1 bis 8, **dadurch gekennzeichnet,** daß die Hub- und Stützelemente (53) gabelförmig ausgebildet sind und für den Verschlußbügel (21a) ein im Drehbereich der Flasche (20) wirksames Gleitstück (53a*) und an einem Gabelast eine rampenartige, den Oberbügel (21a***) untergreifende Steigkurve (53a**) bilden, und daß im Umfangsbereich des Drehtisches (D), in dem nach Verdrehung der Flasche (20) das Gleitstück (53a*) und die Steigkurve (53a**) wirksam sind, optische Prüfmittel (14a,14b) für die Seitenwandkontrolle der Flasche (20) vorgesehen sind.

## Claims

1. An inspection machine for cliplock bottles, comprising a rotary table (4) having disposed at its periphery receiving locations in which the bottle (20) can be clamped axially between a rotatably controlled rotary plate (22) and a centring head (23) lowerable on to the bottle mouth and can be rotated by means of aligning elements (35, 42) into a rotated position with the stopper (21b) lying on the outside, the machine also having lifting and supporting elements (53) which can be applied to the bowed members (21a) of the cliplocks (21) and by means of which, in the zone of an optical testing system (11a - 14b) disposed fixed on the path of the bottles (20) retained in the receiving locations, the stoppers (21b) of the cliplocks (21) can be transferred from their position on the bottle neck into a raised, freely hanging position in which they can be tested by testing means (12a, 12b) of the testing system (11a - 14b), characterized in that the lifting elements (53) engage below and lift the upper bowed member (21a***) and the lower bowed member (21a*), and disposed in the zone of the testing means (12a, 12b) is a finger-like rotating and aligning element (46) which can be moved in between the arms of the raised upper bowed member (21***) and engages with the stopper (21b), which it moves with its rubber seal (21b**) into an outwardly directed position in relation to the testing means (12a, 12b) and retains the stopper (21b) in that position.

2. An inspection machine according to claim 1, characterized in that the rotating and aligning element (46) bears a spring-loaded hold-down device (46 ) which, when the rotating and aligning element (46) is introduced between the arms of the upper bowed member (21a***), acts on said upper bowed member (21a***), which it forces on to the lifting and supporting element (53).

3. A cliplock bottle inspection machine according to claims 1 or 2, characterized in that associated with each lifting and supporting element (53) is a clamping element (35) between which and the lifting and supporting element (53) the upper bowed member (21a** ) supported thereby can be clamped, more particularly at its joint.

4. An inspection machine according to one of claims 1 to 3, characterized in that the finger-like rotating and aligning element (46) has a profiling (46a) which on the introduction of said element rotates the stopper (21b), with the exception of its smooth end face (21b*).

5. An inspection machine according to one of claims 1 to 4, characterized in that associated with each lifting element (53) is a clamping element (35) between which and the lifting element (53) the lower bowed member (21a**) supported thereby can be clamped.

6. An inspection machine according to one of claims 1 to 5, characterized in that the rotating and aligning element (46) bears resiliently against a spring (47a).

7. An inspection machine according to one of claims 1 to 6, characterized in that the lifting elements form for the bowed members (21a*, 21a**) a sliding block (53a*) operative over a range of rotation of 90°, the bottles (20) being rotated by the rotatably controlled rotary plates (22) through 90° when the sliding block (53a*) is operative, and the optical testing system has testing means (13a - 14b) for side wall inspection.

8. An inspection machine according to claim 7, characterized in that the rotatably controlled rotary blades (22) rotate the bottles (20) into a predetermined rotated position in relation to the testing means (13a - 14b) for side wall inspection.

9. An inspection machine according to one of claims 1 to 8, characterized in that the lifting and supporting elements (53) are constructed fork-shaped and form for the lower bowed member (21a) a sliding block (53a*), operative in the range of rotation of the bottle (20), and on one arm of the fork a ramp-like climb curve (53a**) engaging below the upper bowed member (21a***), and in the peripheral area of the rotary table (4) in which after the rotation of the bottle (20) the sliding block (53a*) and the climb curve (53a**) are operative, optical testing means (14a, 14b) are provided for inspecting the side wall of the bottle (20).

## Revendications

1. Machine d'inspection, pour bouteilles à fermeture à arceau, comprenant une table tournante (4) comportant, d'une part, des emplacements de positionnement qui sont disposés à sa périphérie et auxquels les bouteilles (20) peuvent être serrées axialement entre une selle rotative (22), commandée en rotation, et une tête de centrage (23) agencée de façon à pouvoir être abaissée sur le goulot de la bouteille,et peuvent être déplacées en rotation, au moyen d'éléments d'orientation (35, 42), dans une position angulaire dans laquelle le bouchon (21b) de la fermeture est placé vers l'extérieur et, d'autre part, des éléments de poussée et d'appui (53) qui sont agencés de façon à pouvoir être appliqués sur les arceaux (21a) des fermetures à arceau (21) et au moyen desquels, dans la zone d'un dispositif de contrôle optique (11a-14b) disposé d'une manière fixe sur la trajectoire des bouteilles (20) maintenues aux emplacements de positionnement, les bouchons (21b) des fermetures à arceau (21) peuvent être déplacés de leur position sur le col de la bouteille à une position dans laquelle ils sont soulevés et pendent librement et dans laquelle ils peuvent être examinés à l'aide de moyens de contrôle (12a, 12b) du dispositif de contrôle (11a-14b), caractérisée en ce que les éléments de poussée (53) saisissent par-dessous, en les soulevant, l'arceau supérieur (21a***) et l'arceau inférieur (21a*),et en ce que, dans la zone des moyens de contrôle (12a, 12b), il est disposé un élément de déplacement en rotation et d'orientation (46), en forme de doigt, qui vient en prise sur le bouchon (21b) de la fermeture en pouvant être introduit entre les branches de l'arceau supérieur (21a***) soulevé et qui amène le bouchon (21) de la fermeture, par sa garniture d'étanchéité en caoutchouc (21b**), dans une position dans laquelle le bouchon est tourné vers l'extérieur, en direction des moyens de contrôle (12a, 12b), et dans laquelle cet élément (46) le maintient.

2. Machine d'inspection suivant la revendication 1, caractérisée en ce que l'élément de déplacement en rotation et d'orientation (46) porte un organe de serrage vers le bas (46b), à ressort, qui, lors de l'introduction de l'élément de déplacement en rotation et d'orientation (46) entre les branches de l'arceau supérieur (21a***), agit sur cet arceau supérieur (21a***) en l'appliquant sur l'élément de poussée et d'appui (53).

3. Machine d'inspection suivant l'une des revendications 1 et 2, caractérisée en ce qu'à chaque élément de poussée et d'appui (53), il est associé un élément de serrage (35), et en ce que l'arceau de blocage (21a**), qui est soutenu par l'élément de poussée et d'appui (53), peut être serré, notamment à l'endroit de son articulation, entre l'élément de serrage (35) et l'élément de poussée et d'appui (53).

4. Machine d'inspection suivant l'une des revendications 1 à 3, caractérisée en ce que l'élément de déplacement en rotation et d'orientation (46) en forme de doigt comporte une partie profilée (46a) qui, lors de son introduction, agit, en le déplaçant en rotation, sur le bouchon (21b) de la fermeture à l'exception de sa face frontale lisse (21b*).

5. Machine d'inspection suivant l'une des revendications 1 à 4, caractérisée en ce qu'à chaque élément de poussée (53) est associé un élément de serrage (35), et en ce que l'arceau de blocage (21a**), qui est soutenu par l'élément de poussée (53), peut être serré entre l'élément de serrage (35) et l'élément de poussée (53).

6. Machine d'inspection suivant l'une des revendications 1 à 5, caractérisée en ce que l'élément de déplacement en rotation et d'orientation (46) est en appui sur un ressort (47a) de façon à pouvoir céder.

7. Machine d'inspection suivant l'une des revendications 1 à 6, caractérisée en ce que les éléments de poussée constituent une pièce de glissement (53a*) pour les arceaux (21a*, 21a**), cette pièce de glissement agissant dans un domaine angulaire de 90°, de sorte que, lorsque l'élément de glissement (53a*) remplit sa fonction, les bouteilles (20) sont déplacées en rotation de 90° par les selles rotatives (22) commandées en rotation, et en ce que le dispositif de contrôle optique comporte des moyens de contrôle (13a-14b) permettant le contrôle de la surface latérale.

8. Machine d'inspection suivant la revendication 7, caractérisée en ce que les selles rotatives (22) commandées en rotation déplacent les bouteilles (20) en rotation jusqu'à une position angulaire préfixée vis-à-vis des moyens de contrôle (13a-14b) en vue du contrôle de la surface latérale.

9. Machine d'inspection suivant l'une des revendications 1 à 8, caractérisée en ce que les éléments de poussée et d'appui (53) sont en forme de fourche et constituent, pour l'arceau (21a) de la fermeture, une pièce de glissement (53a*) qui remplit sa fonction dans le domaine de déplacement angulaire de la bouteille (20) et, sur une branche de la fourche, une came montante (53a**) qui est en forme de rampe et saisit par-dessous l'arceau supérieur (21a***), et en ce que, dans la zone périphérique de la table tournante (D) dans laquelle la pièce de glissement (53a*) et la came montante (53a**) remplissent leur fonction après un déplacement en rotation de la bouteille (20), il est prévu des moyens de contrôle optique (14a, 14b) pour le contrôle de la surface latérale de la bouteille (20).
